(19) Europäisches Patentamt
European Patent Office
Office européen des brevets

(11) Publication number: **0 000 322**
**B1**

(12) **EUROPEAN PATENT SPECIFICATION**

(45) Date of publication of patent specification: 05.05.82

(21) Application number: 78850006.4

(22) Date of filing: 03.07.78

(51) Int. Cl.³: C 07 C 87/28, C 07 C 87/29, C 07 C 93/14, C 07 D 213/38, A 61 K 31/135, A 61 K 31/44

# (54) Compounds having an anti-depressive or tranquilizing activity, pharmaceutical compositions containing them, and processes and intermediates for their preparation.

(30) Priority: 04.07.77 GB 2799277
31.05.78 GB 2799277
22.05.78 GB 2124978

(43) Date of publication of application:
10.01.79 Bulletin 79/1

(45) Publication of the grant of the patent:
05.05.82 Bulletin 82/18

(84) Designated Contracting States:
BE CH DE FR LU NL SE

(56) References cited:
GB - A - 624 117
GB - A - 956 616
FR - A - 1 021 301
FR - A - 1 160 995
FR - A - 1 226 401

CHEMICAL ABSTRACTS, vol. 82, (1975) nr. 170039c
As intermediate: 3,3-di-(4-fluorophenyl)-1-methyl-propylamine

(73) Proprietor: Astra Läkemedel Aktiebolag
Strängnäsvägen 44
S-151 85 Södertälje (SE)

(72) Inventor: Carnmalm, Bernt Sigfrid Emanuel
Törnrosavägen 14
S-151 52 Södertälje (SE)
Inventor: Högberg, Thomas
Vallmostigen 5
S-150 20 Järna (SE)
Inventor: de Paulis, Tomas
Villavägen 13
S-150 11 Björnlunda (SE)
Inventor: Ross, Svante Bertil
Hedvägen 8
S-151 52 Södertälje (SE)

(74) Representative: Wurm, Bengt Runio et al,
S-151 85 Södertälje (SE)

EP 0 000 322 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Compounds having an anti-depressive or tranquilizing activity, pharmaceutical compositions containing them, and processes and intermediates for their preparation

The present invention is related to new compounds of the diarylalkylamine type having thereapeutic activity, to methods for preparing such compounds, to pharmaceutical preparations comprising such compounds and to methods of treatment employing such compounds.

The object of the invention is to obtain compounds having a therapeutical activity in the central nervous system, especially an anti-depressive or a tranquilizing activity.

Prior Art

British Patent 1,429,068 discloses compounds corresponding to the general formula:

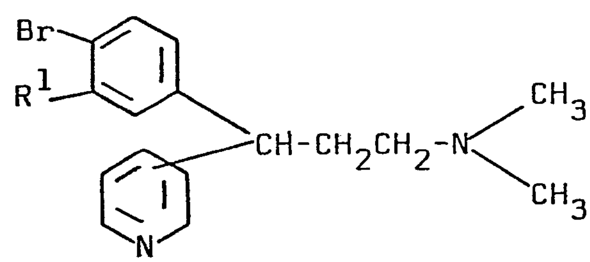

having anti-depressive activity. Belgian Patent 835,802 discloses compounds of the general formula:

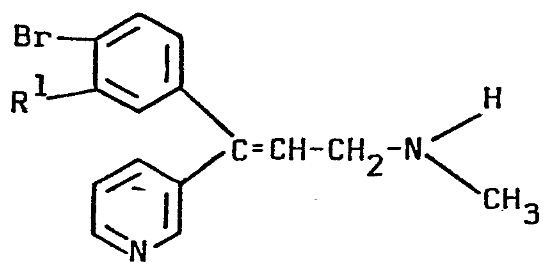

having anti-depressive activity.

Compounds following within the general formula

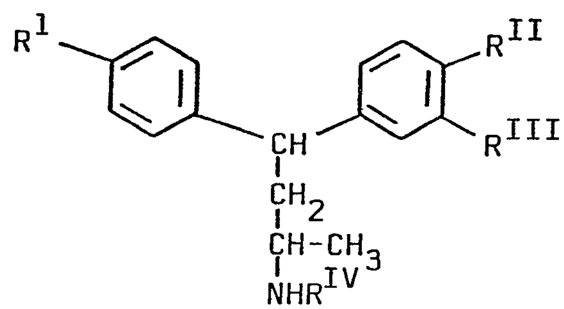

are disclosed by prior publications as follows: $R^I=CH_3O$, $R^{II}=OH$, $R^{III}=R^{IV}=H$, and $R^I=Cl$, $R^{II}=OH$, $R^{III}=CH_3$, $R^{IV}=H$ having hypotensive properties, by British Patent 765,881: $R^I=R^{II}=R^{III}=R^{IV}=H$ by French Patent 2,215,973: $R^I=R^{II}=R^{III}=H$, $R^{IV}$=tertiary butyl having spasmolytic properties, by British Patent 923,942; $R^I=R^{II}=R^{III}=H$, $R^{IV}=CH_3$ having spasmolytic properties, by US Patent 2,446,522.

South African Patent 62/4154 discloses i.a. a compound having the formula

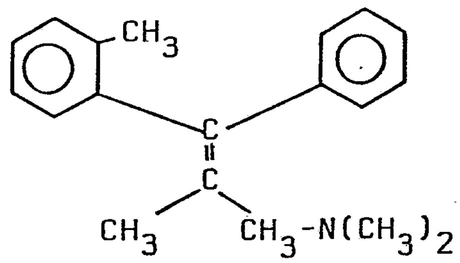

claimed to have a therapeutic utility especially as an antitussive.

In J. Med. *14* 161 (1971) a compound of the formula

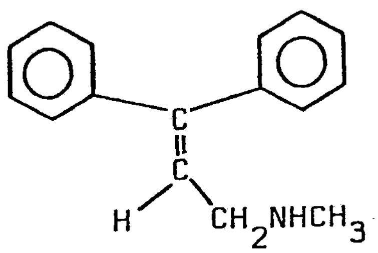

is disclosed as an antidepressant.

Chemical Abstract vol. 82 (1975) 170339c discloses the compound of the formula

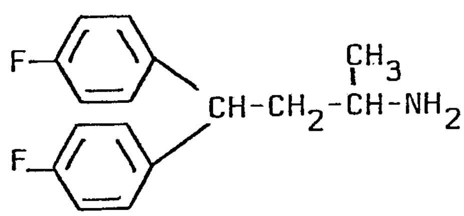

as a chemical intermediate.

Related patent application

Certain intermediates disclosed in the present specification are claimed in a European divisional patent application having no. 80105028.7.

General Outline of the Invention

According to the present invention it has been found that compounds of the general formula below have advantageous, therapeutic properties:

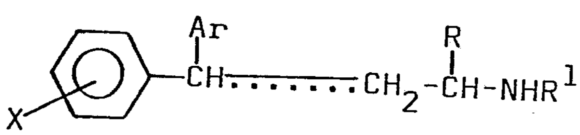

I

In these compounds the dotted line represents an optional double bond. These compounds may be divided into two groups defined by the formulae

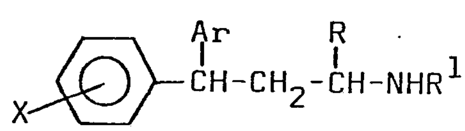

Ia

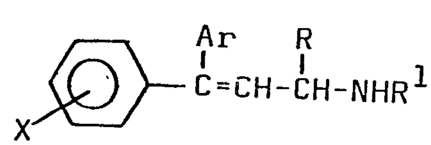

Ib

In the compounds of formula I Ar represents the group

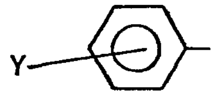

wherein Y is bound in the 2-, 3-, or 4-position and represents a lower alkyl group, a lower alkoxy group, a halogen, a trifluoromethyl group, or an amino or a mono- or di-lower alkyl amino group, or Ar represents a pyridyl group bound in the 2-, 3- or 4-position, X represents hydrogen, a lower alkyl group, a lower alkoxy group, a halogen, a trifluoromethyl group, an amino group or a mono- or di-lower alkyl amino group, R is a lower alkyl group and $R^1$ is hydrogen or a lower alkyl group. By lower alkyl and alkoxy groups are meant groups comprising up to 3 carbon atoms. Halogen may be any of the elements F, Cl, Br or I. Therapeutically acceptable salts of the compounds of the invention as well as differently hydrated or anhydrous forms of such compounds or salts are within the scope of the invention.

The compounds of formula Ia above wherein Ar is identical to the group

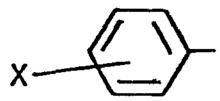

contain one asymmetric carbon atom. The remaining compounds of formula Ia contain two asymmetric carbon atoms and can therefore exist in two diastereomeric forms which can be separated by methods known in the art. Further the compounds of formula Ia above may be resolved into their optical enantiomers by using optically active acids such as i.a. tartaric acid, mandelic acid, dibenzoyl tartaric acid as known in the art. The compounds of the invention may be used as mixtures of diastereomeric forms or as racemic mixtures of the pure diastereomers or as the pure enantiomers mentioned above. The therapeutic properties may reside to a greater or lesser extent in one of the enantiomers or mixtures mentioned above.

Due to the lack of free rotation in the double bond the compound of formula Ib in which the group Ar is not identical with the group

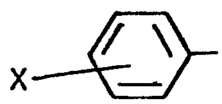

may exist in different stereoisomeric forms, that is in cis-trans isomers or, according to the IUPAC nomenclature (J. Org. Chem. *35*, 2849—2867, Sept. 1970), in an E-form and a Z-form. The compound may be used therapeutically as a mixture of geometrical isomers or in pure E or Z form. The pure geometrical isomers may be prepared from an isomer mixture, from an isomer-pure starting material or directly by a stereoselective synthesis.

It should be noted that in the IUPAC nomenclature compounds of formula Ib in the form of pure geometrical isomers which are similar in structure may be named the E-form for one subgroup of compounds and the Z-form for another subgroup. The two structural formulas below illustrate this fact.

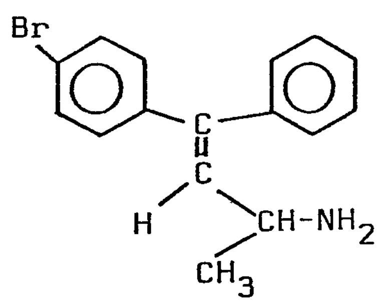

E-form

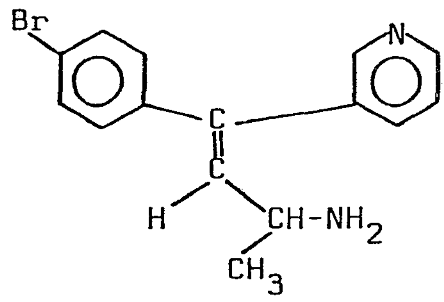

Z-form

All the compounds of formula Ib further contain one asymmetric carbon atom. The compounds of formula Ib may be resolved into their optical enantiomers by using optically active acids such as i.a. tartaric acid, mandelic acid, dibenzoyl tartaric acid as known in the art. The compounds of formula Ib may be used as mixtures especially racemic mixtures, or as the pure enantiomers of the geometrical isomers mentioned above. The therapeutic properties may reside to a greater or lesser extent in one of the enantiomers or mixtures mentioned above.

The invention relates in one aspect to pharmaceutical compositions comprising as active ingredient a therapeutically effective amount of a compound of the formula I.

The invention relates also to compounds of the formula I for use in the treatment of depressions and for use in the treatment of anxiety.

The invention also relates to such compounds per se of the formula I wherein X is hydrogen.

The compounds of the invention show an activity in the central nervous system which makes them useful as neuropharmacological agents for treatment of various diseases in animals including man. The compounds are expected to be especially useful as anti-depressive, anxiolytic or tranquilizing agents in man.

Preferred Embodiment of the Invention

Of the compounds of the invention defined by formula I above those wherein Ar is

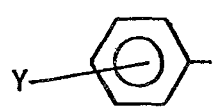

are to be specially mentioned. Of those the compounds wherein X is hydrogen are preferred and especially those wherein Y is F, Br or $CH_3O$—.

As preferred individual compounds should be mentioned:

(β)-3-(4-fluorophenyl)-1-methyl-3-phenylpropylamine,
(β)-3-(4-bromophenyl)-1-methyl-3-phenylpropylamine,
(α)-3-(4-methoxyphenyl)-1-methyl-3-phenylpropylamine, and
(β)-3-(3-bromophenyl)-1-methyl-3-phenylpropylamine

being non-selective inhibitors of neuronal noradrenaline and 5-hydroxytryptamine uptake;

(α)-3-(2-bromophenyl)-1-methyl-3-phenylpropylamine,
(E)-3-amino-1-(3-bromophenyl)-1-phenylbutene, and
(Z)-3-amino-1-(3-bromophenyl)-1-phenylbutene

being selective inhibitors of neuronal noradrenaline uptake; and

3-amino-1,1-di-(4-methoxyphenyl)-1-butene,
3,3-di-(4-fluorophenyl)-1-methylpropylamine,
(β)-3-(4-methoxyphenyl)-1-methyl-3-phenylpropylamine, and
(E)-3-amino-1-(4-bromophenyl)-1-phenylbutene,

being selective inhibitors of neuronal 5-hydroxytryptamine uptake; as well as salts and precursors of said compounds. The neuronal uptake mechanism are discussed further in the chapter "Pharmacological evaluation" below.

Generally preferred in all classes of the compounds of the invention are those wherein $R^1$ represents hydrogen and R represents a methyl group.

Methods of Preparation

The compounds of the formula I may be prepared by

a) reducing a compound of the formula

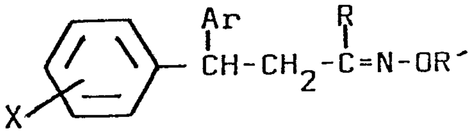

II

wherein Ar, X and R have the meaning defined above and R′ is a hydrogen atom or an alkyl, an acyl or an alkylsulfonyl group having 1—3 carbon atoms, to the obtention of a compound of formula Ia in which $R^1$ is hydrogen, and if desired converting this primary amine to a secondary amine in a manner known in the art. The reduction may be carried out by known methods e.g. employing a hydride reagent such as lithium aluminium hydride,

b) preparing a reactive ester of an alcohol of the formula

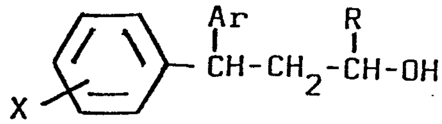

III

wherein Ar, X and R have the meaning defined above and reacting the ester obtained with an amine of the formula $NH_2R^1$, wherein $R^1$ has the meaning defined above. The reactive ester may be obtained by treating the alcohol with a halogenating agent such as thionyl chloride, thionyl bromide or phosphorus tribromide, or with an arylsulphonyl halide such as p-toluenesulphonyl chloride,

c) reducing a compound of the formula

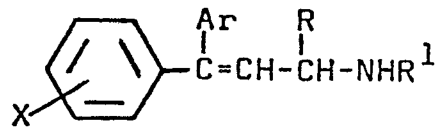

Ib

wherein Ar, X, R and $R^1$ have the meaning defined above. The reduction may be carried out by methods known in the art e.g. by catalytic hydrogenation using catalysts such as Raney nickel, palladium on charcoal, platinum dioxide or rhodium,

d) reacting a ketone of the formula

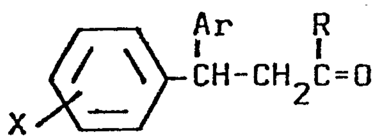

IV

wherein Ar, X and R have the meaning defined above, with ammoniumformate or methylammonium-formate according to Leuckart-Wallach. The formate may be added as such, or obtained by formation *in situ* from formamide or methyl formamide, or from formic acid and ammonia, or methylamine.

The intermediates of formulae II, III, and IV above are novel.

The intermediates of formula II may be prepared by reacting the ketone of the formula IV above a hydroxylamine derivative of formula $NH_2OR^1$, wherein $R^1$ has the meaning defined above.

The intermediate of formula III may be prepared by hydride reduction of the compound of formula IV which, in turn, may be obtained by

1) reacting an alpha, beta-unsaturated ketone of the formula

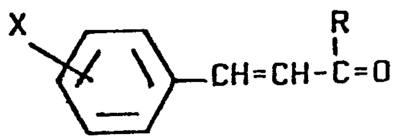

V

wherein X and R have the meaning defined above, with a metal-organic reagent, such as magnesium, lithium or sodium derivative of an arylhalide of the formula Ar—Y′, wherein Ar has the meaning defined above and Y′ is a chlorine, bromine or iodine atom, in the presence of catalytic amounts of cuprous ions,

2) reacting a diarylcarbinol of the formula

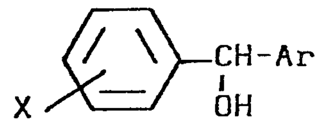

VI

wherein Ar and X have the meaning defined above, first with thionyl chloride, then with ethyl acetoacetate in the presence of suitable condensation catalyst such as sodium acetate or the like.

The compounds of formula Ia are preferably prepared by method a). The reaction according to method a) is preferably performed in diethyl ether with a slight excess of lithium aluminium hydride under inert atmosphere.

The new compounds of formula Ia may be used therapeutically as the racemic mixtures of (+)- and (—)-forms, which in the usual case are obtained at the synthesis. Isomer mixtures obtained may be resolved by methods known *per se* into corresponding optically active modifications. If desired, the optically active modifications may be prepared by way of direct synthesis, e.g. via an optically active compound as described above.

The compounds of the formula I in the invention may be prepared by:

e) Dehydration of a carbinol of the formula

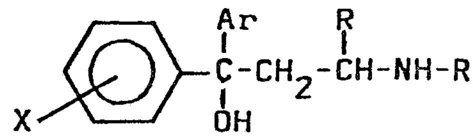

VII

to a compound of formula Ib.

The dehydration of the starting material may be done by means of treatment with hydrochloric acid HCl and heating of the reaction mixture. The dehydration of the starting material may also be done by means of other types of acid-catalysis, such as by means of sulfuric acid $H_2SO_4$, phosphoric acid $H_3PO_4$, potassium hydrogen sulphate $KHSO_4$, or oxalic acid $(COOH)_2$. Other methods for the dehydration of the starting material to the formation of a compound of the formula I are dehydration using phosphoroxichloride $POCl_3$ in pyridine, and dehydration with thionylchloride, $SOCl_2$, in pyridine. Also a catalytic dehydration of the starting material may be used. The dehydration is in this case carried out at a temperature of about 300 to 500°C using a catalyst such as kaolin, alumina or aluminium oxide.

f) Reaction of a compound of the formula

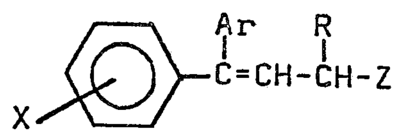

VIII

wherein Z is a leaving group such as F, Cl, Br, I, $OSO_2R^1$, wherein $R^1$ is alkyl, aralkyl or aryl, with an amine of the formula $NH_2R^1$ or with a derivative thereof such as hexamethylenetetraamine, alkali phthalimide, lithium bisbenzenesulfenamide, guanidine, sodium cyanate, sodium azide, a carboxamide, or a sulfonamide. When an amine derivative is alkylated the product obtained is subsequently hydrolyzed or in some other way converted into a primary or secondary amine of formula Ib. A preferred amine derivative is potassium phthalimide.

This reaction is also useful for preparation of the compounds of formula Ia by employing as a starting material a saturated compound corresponding to the compound of formula VIII.

g) Oxidation (halogenation) of the benzylic carbon atom of a compound of formula IX

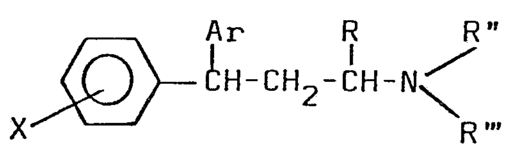

IX

e.g. with N-bromosuccinimide, R″, R‴ being protective groups for the amino function preferably joined as the group

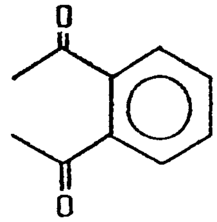

or alternatively corresponding to one of the amine derivatives specified under f) above, followed by elimination of the group formed by oxidation at the benzyl carbon atom, giving a compound of the formula

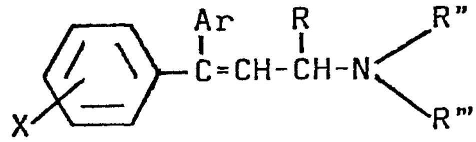

X

which is transformed into the compound of formula Ib by splitting off the groups R'' and R''', e.g. by hydrolysis or hydrazinolysis. In this manner it is possible to synthesize the compounds of formula Ib from the corresponding saturated compounds of formula Ia, by introducing in those compounds protective groups R'' and R''' in a known manner to the obtention of a compound of formula IX.

The intermediates of formulae VII, VIII, IX and X above are novel.

The intermediate of formula VII may be obtained by preparing a Grignard or lithium compound from a halobenzene of the formula

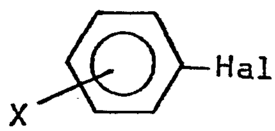

Hal = Br, Cl

and reacting it with an ester of the formula

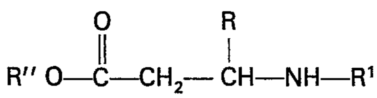

wherein R'' is an alkyl, aralkyl or aryl group.

The intermediate formula VIII may be isolated (cf. Example 15) and subsequently dehydrated or alternatively the crude product in the preparation of formula VII may be dehydrated directly (cf. Example 17).

The intermediate of formula VIII may be obtained from a compound of formula

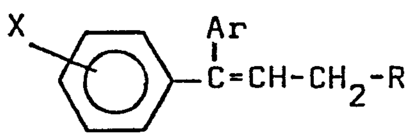

XI

wherein X, Ar and R have the meaning defined above, by oxidation of the allylic carbon to the formation of a compound of the formula

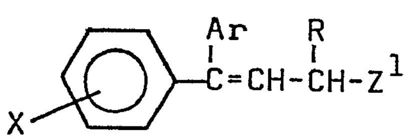

III:1

wherein $Z^1$ represents Z or OH, e.g. by reaction with N-bromosuccinimide ($Z^1$ = Br) or selenium dioxide ($Z^1$ = OH). The latter compound may be transformed to a reactive derivative III by treatment with an agent such as $SOCl_2$, $SOBr_2$ or $PBr_3$ or with $ClSO_2R^1$.

Pharmaceutical Preparations

In clinical practice the compounds of the formula I will normally be administered orally, rectally or by injection, in the form of pharmaceutical preparations comprising the active ingredient either as a free base or as a pharmaceutically acceptable non-toxic, acid addition salt, e.g. the hydrochloride, hydrobromide, lactate, acetate, phosphate, sulphate, sulphamate, citrate, tartrate, oxalate and the like in association with a pharmaceutically acceptable carrier. Accordingly, terms relating to the compounds of the formula I whether generically or specifically are intended to include both the free amine base and the acid addition salts of the free base, unless the context in which such terms are used, e.g. in the specific examples would be inconsistent with the broad concept. The carrier may be a solid, semisolid or liquid diluent or capsule. These pharmaceutical preparations constitute a further aspect of this invention. Usually the active substance will constitute from 0.1 to 99% by weight of the preparation, more specifically between 0.5 and 20% by weight for preparations intended for injection and between 2 and 50% by weight for preparations suitable for oral administration.

To produce pharmaceutical preparations containing a compound of the formula I in the form of dosage units for oral application the selected compound may be mixed with a solid pulverulent carrier, e.g. lactose, saccharose, sorbitol, mannitol, starches such as potato starch, corn starch or amylopectin, cellulose derivatives, a binder such as gelatine or polyvinyl-pyrrolidone, and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatine, talcum, titanium dioxide, and the like. Alternatively, the tablet can be coated with a lacquer in a readily volatile organic solvent or mixture of organic solvents. Dyestuff may be added to these coatings in order to readily distinguish between tablets containing different active substances or different amounts of the active compounds.

For the preparation of soft gelatine capsules (pearl shaped closed capsules) consisting of gelatine and for example, glycerol or similar closed capsules, the active substance may be admixed with a vegetable oil. Hard gelatine capsules may contain granulates of the active substance in combination with solid, pulverulent carriers such as lactose, saccharose, sorbitol, mannitol, starches (e.g. potato starch, corn starch or amylopectin), cellulose derivatives or gelatine.

Dosage units for rectal application can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example, solutions containing from about 0.2% to about 20% by weight of the active substance herein described, the balance being sugar and a mixture of ethanol, water, glycerol, and propyleneglycol. Optionally such liquid preparations may contain colouring agents, flavouring agents, saccarine and carboxymethylcellulose as a thickening agent.

Solutions for parenteral applications by injection can be prepared in an aqueous solution of a water-soluble pharmaceutically acceptable salt of the active substance preferably in a concentration of from about 0.5% to about 10% by weight. These solutions may also contain stabilizing agents and/or buffering agents and may conveniently be provided in various dosage unit ampoules.

Suitable daily doses of the compounds of the formula I in therapeutic treatment is 25 to 250 mg for peroral administration, preferably 50 to 150 mg, and 5 to 50 mg for parenteral administration preferably 10 to 30 mg. A preparation in dosage unit form for oral administration may contain 10 to 50 mg, preferably 10 to 25 mg of active substance per dosage unit.

*Working Examples*

Example 1

Preparation of 4-(3-bromophenyl)-4-phenylbutan-2-one

To 2.4 g (0.10 mol) of magnesium turnings, covered with 20 ml of anhydrous diethyl ether and treated with some crystals of iodine under nitrogen atomosphere, 23.5 g (0.10 mol) of 1,3-dibromobenzene in 80 ml of ether was added. The rate of addition was adjusted, to maintainn a gentle reflux of the solvent. When the Mg turnings had disappeared (30 min) 0.75 g (0.005 mol) of cuprous bromide was added and the mixture was stirred for 10 min at room temperature. A solution of 13.6 g (0.09 mol) of benzalacetone in 100 ml of ether was added dropwise at +10°C. Then the reaction mixture was allowed to reach room temperature for 2 hours. The mixture was poured into 400 ml of 10% aqueous solution of ammonium chloride, the aqueous layer was separated and the product was extracted with 2 × 200 ml of ether. The ethereal layer was washed with water, dried with $Na_2SO_4$ and the solvent was evaporated. The crude 4-(3-bromophenyl)-4-phenylbutan-2-one obtained (26.5 g, 0.086 mol) was added to a solution of 20 g (0.28 mol) of hydroxylamine hydrochloride in 300 ml of ethanol and 100 ml of anhydrous pyridine. The mixture was heated under reflux for 4 hours. After cooling the solvent was evaporated *in vacuo* and the ketoxime was extracted with ether from an aqueous solution. Washing of the extract with water and drying followed by evaporation of the solvent gave 27.5 g of 4-(3-bromophenyl)-4-phenylbutan-2-one oxime.

Example 2

Preparation of 3-(3-bromophenyl)-1-methyl-3-phenylpropylamine oxalate. (Method a)

To the oxime obtained according to Example 1 (27.5 g) 100 ml of carbon tetrachloride was added and evaporated twice in order to remove traces of water. The residue was dissolved in a mixture of 300 ml of anhydrous ether and 150 ml of anhydrous tetrahydrofuran. To the stirred mixture 3.5 g (0.0086 mol) of lithium aluminium hydride was added in portions under nitrogen atmosphere at room temperature. The reaction mixture was stirred for 8 hours. Then 25 ml of 2M NaOH was dropwise added and the precipitated inorganic salts were removed by filtration. The filtrate was shaken with 3 x 300 ml of 1 M HCl and the combined aqueous layers were made alkaline by addition of 35 ml of 30% NaOH. Extraction with 3 x 200 ml of methylene chloride, washing, drying and evaporation of the solvent gave 8.9 of the primary amine as an oil. To a hot solution of 7.9 g (0.026 mol) of the amine in 100 ml of isopropylalcohol 1.1 g (0.014 mol) of oxalic acid in 10 ml of ethanol was added. 6.2 g of the diamine oxalate was collected. Recrystallization from 160 ml of a mixture of ethanol and isopropylalcohol (1:1) yielded 4.43 g, mp 134—138°C.

Analysis: C calcd 58.5%, found 58.7%; H calcd 5.48%, found 5.80%; N calcd 4.01%, found 4.07%.

Example 3

Separation of 3-(bromophenyl)-1-methyl-3-phenylpropylamine into its diastereomers

The free amine (2.9 g, 0.009 mol) obtained from 3.8 g of the oxalate prepared according to Example 2, was dissolved in 40 ml of ethyl acetate. A hot solution of 1.1 g (0.009 mol) of maleic acid in 20 ml of ethanol was added. There was obtained 1.3 g of the maleate. Recrystallization from 18 ml of isopropylalcohol gave 0.65 g of the pure alpha isomer, mp 163—165°C. The high field part of the NMR spectrum ($COCl_3$) displayed a triplet at 4.1 ppm (J=7.8 Hz), a quartet at 2.8 ppm (J=6.2 Hz), a double dublet at 2.0 ppm (two protons) and a doublet at 1.5 ppm (J=6.1 Hz) (three protons).

Analysis: C calcd 57.15%, found 57.45%; H calcd 5.28%, found 5.35%; Br calcd 19.1%, found 19.05%; N calcd 3.33%, found 3.20%; O calcd 15.23%, found 15.00%.

The solvents of the first mother liquors of the diamine oxalate prepared according to example 2 were evaporated and the residue was extracted with ether from an alkaline solution. There was obtained 1.2 g of free amine. The fumarate was prepared in ethyl acetate from half an equivalent of fumaric acid and recrystallized twice from acetonitrile-isopropylalcohol affording 0.28 g of the pure beta isomer as the diamine fumarate, mp 184—186°C.

Analysis: C calcd 59.7%, found 60.3%; H calcd 5.6%, found 5.7%; N calcd 3.9%, found 3.7%.

Example 4

($\alpha$)-3-(4-fluorophenyl)-1-methyl-3-phenylpropylamine oxalate, mp 186—188°C (EtOH—EtOAc, 1:1) and ($\beta$)-3-(4-fluorophenyl)-1-methyl-3-phenylpropylamine hydrochloride, mp 171—172°C (EtOAc) were prepared from 4-(3-fluorophenyl)-4-phenylbutan-2-one oxime in accordance with Examples 2 and 3.

Example 5

($\alpha$)-3-(4-bromophenyl)-1-methyl-3-phenylpropylamine maleate, mp 168—170°C (EtOH—EtOAc, 1:1) and ($\beta$)-3-(4-bromophenyl)-1-methyl-3-phenylpropylamine maleate, mp 161—162°C (i-PrPH—EtOAc, 3:1) were prepared from 4-(4-bromophenyl)-4-phenylbutan-2-one oxime in accordance with Examples 2 and 3.

Example 6

($\alpha$)-3-(4-methoxyphenyl)-1-methyl-3-phenylpropylamine maleate, mp 146—148°C (i-PrOH) and ($\beta$)-3-(4-methoxyphenyl)-1-methyl-3-phenylpropylamine maleate, mp 124—133°C (EtOAc) were prepared from 4-4-methoxyphenyl)-4-phenylbutan-2-one oxime in accordance with Examples 2 and 3.

Example 7

($\alpha$)-1-methyl-3-(4-trifluoromethylphenyl)-3-phenylpropylamine maleate, mp 165—166°C and ($\beta$)-1-methyl-3-(4-trifluoromethylphenyl)-3-phenylpropylamine maleate, mp 165—167°C were prepared from 4-(4-trifluoromethylphenyl)-4-phenylbutan-2-one oxime in accordance with Examples 2 and 3.

Example 8

Preparation of 4-(2-bromophenyl)-4-phenylbutan-2-one

To a solution of 24.3 g (0.13 mol) of 2-bromobenzaldehyde in 80 ml of acetone, 1.0 ml of 10 M NaOH was slowly added at 0°C. The reaction mixture was allowed to reach room temperature and stirred for another 2 hours. Then it was poured into 400 ml of water, to which 10 ml of 2 M HCl had been added. Extraction with ether, drying and evaporation of the solvent gave 18.9 g of 2-bromobenzalacetone as an oil. This was dissolved in 150 ml of ether and added to a Grignard reagent

prepared from 1.1 g (0.045 mol) of magnesium turnings 6.6 g (0.042 mol) of bromobenzene and 0.2 g of CuBr in 150 ml of ether. The mixture was stirred under nitrogen atmosphere for 2 hours. It was poured into 450 ml of ice-water to which 18 g of ammonium chloride had been added. Extraction with ether gave 11.0 g of the desired ketone as an oil.

Example 9

($\alpha$)-3-(2-bromophenyl)-1-methyl-3-phenylpropylamine maleate, mp 145—146°C (i-PrOH) and ($\beta$)-3-(2-bromophenyl)-1-methyl-3-phenyl-propylamine maleate, mp 135—137°C (EtOH—EtOAc, 1:4) were prepared from 4-(2-bromophenyl)-4-phenylbutan-2-one oxime in accordance with Examples 2 and 3.

Example 10

Preparation of N,1-dimethyl-3-(4-bromophenyl)-3-phenylpropylamine

The free base of 3-(4-bromophenyl)-1-methyl-3-phenylpropylamine (0.07 g, 0.0023 mol) was dissolved in 50 ml of chloroform. 1.2 ml (0.0024 mol) of 2 M NaOH and 0.25 g (0.0024 mol) of ethyl chloroformate were added separately and dropwise with vigorous stirring at 15°C. Stirring was continued for 2 hours at room temperature.

Then 25 ml of water was added and the organic layer was separated, dried, and the solvent was evaporated to give 1.0 g of N-ethoxycarbonyl-3-(4-bromophenyl)-1-methyl-3-phenylpropylamine as a colourless oil.

Treatment of the carbamate with 0.25 g (0.006 mol) of $LiAlH_4$ in 60 ml of ether for 14 hours gave 0.20 g of the secondary amine after extraction with ether/hydrochloride and NaOH/ether. The hydrochloride was prepared and recrystallized from 18 ml of acetone to give 0.12 g, mp 146—148°C.

The oxalate had mp 106—111°C from acetone.

Example 11

Preparation of 3-(3-bromophenyl)-1-methyl-3-phenylpropylamine. (Method d)

A mixture of 22.1 g (0.073 mol) of 4-(3-bromophenyl)-4-phenylbutan-2-one and 230 ml of formamide was heated for 8 hours at 180°C. After cooling water was added and the product was taken up in ether. Drying and evaporation of the solvent gave 30.5 g of a residue.

To this residue 85 ml of conc. hydrochloric acid was added and the mixture was heated under refluxing conditions for 3 hours. Water was added and the non basic materials were removed by shaking the reaction mixture with 100 ml of ether. The aqueous layer was separated and made alkaline by addition of 120 ml of 10 M NaOH. Extraction with 3 x 200 ml of ether, drying ($Na_2SO_4$) and evaporation of the solvent gave 12.9 g of the desired amine.

The maleate was prepared by addition of a hot ethanolic solution of 4.9 g of maleic acid into a warm solution of the amine in 100 ml of ethyl acetate. Recrystallization from EtOH—EtOAc gave 8.4 g of the maleate, mp 158—161°C.

Analysis: C calcd 57.2%, found 57.5%; H calcd 5.28%,found 5.35%; Br calcd 19.0%, found 19.1%; N calcd 3.33%, found 3.20%; O calcd 15.2%, found 15.0%.

Example 12

Preparation of 3,3-di-(4-fluorophenyl)-1-methylpropylamine hydrochloride (Method c)

3,3-di-(4-fluorophenyl)-1-methylallylamine (0.9 g, 0.003 mol) was dissolved in 100 ml of ethanol and transferred to a Parr hydrogenation flask. 1.0 ml of concentrated hydrochloric acid was added followed by 0.2 g of 5% palladium on charcoal. The hydrogenation was effected at a pressure of 3.9 atm for 5.5 hours. The reaction mixture was filtered to remove the catalyst and the solvent of the filtrate was evaporated. Crystallization from EtOAc-i-$Pr_2O$ gave 0.75 g of the desired product, mp 225—229°C.

Analysis: C calcd 64.5%, found 64.5%; H calcd 6.09%, found 6.11%; Cl calcd 11.9%, found 11.8%; F calcd 12.8%, found 12.7%; N calcd 4.70%, found 4.55%.

By the same method there were prepared from the appropriate allylamines:

Example 13

By the method of Example 12 3,3-di-(4-methoxyphenyl)-1-methyl-propylamine fumarate, mp 153—157°C, (EtOH-i-$Pr_2O$) was prepared from the corresponding allylamine.

Example 14

By the method of Example 12 1,3′,3″-trimethyl-3,3-diphenyl-propylamine oxalate, mp 214—215°C, (EtOH—EtOAc) was prepared from the corresponding allylamine.

Example 15

Preparation of 4,4-di-(4-bromophenyl)-4-hydroxy-2-butylamine oxalate

A solution of 81.5 g (0.35 mol) 1,4-dibromobenzene in 500 ml of diethyl ether was added to a stirred mixture of 8.3 g (0.35 mol) magnesium turnings in 25 ml diethyl ether at such a rate that reflux

was maintained. After an additional stirring for 1.5 hours at room temperature the mixture was cooled in an ice-bath and a solution of 11.3 g (0.11 mol) of ethyl 3-amino-butyrate in 25 ml of diethyl ether was added during 15 min.

The mixture was stirred for 1.25 hours at ice-cooling and then for 2.5 hours at reflux. A cold aqueous solution of 25 g ammonium chloride was slowly added, and after stirring the mixture was extracted twice with ether. The ethereal layer was dried over sodium sulphate and the amine was precipitated (10.9 g, 20% yield) with oxalic acid dissolved in ether. M.p. 191—194°C.

Elemental analysis: $C_{18}H_{19}Br_2NO_5$: Found: C 44.5, H 4.0, N 2.7, and O 16.8%. Calculated: C 44.20, H 3.91, N 2.86 and O 16.35%.

## Example 16

Preparation of 3-amino-1,1-di(4-bromophenyl)-1-butene hydrochloride (Method e)

A solution of 3.8 g 4,4-di(4-bromophenyl)-4-hydroxy-2-butylamine oxalate, 25 ml acetic acid and 5 ml conc. aqueous hydrogen chloride was heated under reflux for 30 min. The solvent was evaporated and the residue was made alkaline with sodium hydroxide and extracted twice with ether. The ethereal layer was dried over sodium sulphate and the solvent was evaporated. Acetronitrile and hydrogen chloride in ether were added and the hydrochloride of the title compound (1.7 g) was obtained after recrystallization from acetonitrile/ether. M.p. 216—220°C.

Elemental analysis: $C_{16}H_{16}Br_2ClN$; Found: C 46.7, H 3.9, Cl 8.8 and N 3.1%. Calculated: C 46.02, H, 3.86, Cl 8.49 and N 3.35%.

## Example 17

Preparation of 3-amino-1,1-di(4-methoxyphenyl)-1-butene fumarate (Method e)

A solution of 58.0 g (0.31 mol) of 4-bromoanisole in 300 ml diethyl ether was added dropwise to a stirred mixture of 7.78 g (0.32 mol) magnesium turnings in 250 ml of diethyl ether during 2 hours. The mixture was stirred at room temperature for another 1.5 hours and then heated under reflux for 1.5 hours. The mixture was cooled in an ice-bath and a solution of 10.3 g (0.10 mol) ethyl 3-aminobutyrate in 25 ml diethyl ether was added during 20 min. After stirring over night at room temperature an aqueous solution of 16.6 g (0.31 mol) ammonium chloride was slowly added. The mixture was stirred and then made alkaline and filtered. After separation of the ether phase the aqueous phase was extracted with ether. The combined ethereal layers were extracted twice with 2 M hydrogen chloride. The aqueous phase was made alkaline and extracted with ether and dried over sodium sulphate. After evaporation of the solvent 10.1 g of yellow oil was obtained, which was dissolved in 75 ml of acetic acid and 15 ml of conc. aqueous hydrogen chloride.

The solution was heated under reflux for 30 min and then the solvent was evaporated. The residue was made alkaline and extracted with ether. The ethereal layer was washed with water and extracted with 0.5 M hydrogen chloride. The aqueous phase was washed with ether, made alkaline and extracted with ether. After drying over sodium sulphate the ether was evaporated to give 5.9 g of the title compound as an oil in 21% yield.

The amine was converted to the fumarate, which was recrystallized twice from ethanol/ethyl acetate/hexane to give 6.2 g (17% yield) of the fumaric acid salt in the form $C_{18}H_{21}NO_2 \cdot 3/4C_4H_4O_4$. M.p. 167—167.5°C.

Elemental analysis: $C_{21}H_{24}NO_5$: Found: C 67.8, H 6.52, N 3.89 and O 21.5%.Calculated: C 68.09, H 6.53, N 3.78 and O 21.60%.

## Example 18

3-amino-1,1-di-(4-fluorophenyl)-1-butene fumarate was prepared according to Example 17. M.p. 225—231°C.

## Example 19

3-amino-1,1-di-(3-methylphenyl)-1-butene hydrochloride was prepared according to Example 17. M.p. 216—217.5°C.

## Example 20

Preparation of 1-(4-bromophenyl)-1-phenyl-butene

Sodium dimethylsulfoxide in DMSO, prepared by heating 3.8 g (0.08 mol) sodium hydride (50% in oil) in 100 ml dimethylsolfoxide at 80°C for 40 min, was mixed with 27.0 g (0.07 mol) propyltriphenylphosphonium bromide, prepared by heating propylbromide and triphenylphosphine in toluene at reflux temperature for 14 hours. The mixture was stirred under nitrogen atmosphere at room temperature for 1.5 hours. THen a solution of 13.1 g (0.05 mol) of 4-bromobenzophenone, in a mixture of 100 ml dimethylsulfoxide and 100 ml of anhydrous tetrahydrofuran was added at room temperature. The reaction mixture was stirred for 2 hours, then it was poured into 1,000 ml of ice-water. The product was extracted with 3 x 300 ml of ether, the combined ethereal layer was washed with 100 ml of water. Drying ($Na_2SO_4$) and evaporation of the solvent gave 18 g of oily residue. After trituration with 100 ml of diisopropylether crystal of triphenylphosphine oxide was separated by filtration. Distillation of

the filtrate at 4 Pa gave 12.2 of 1-(4-bromophenyl)-1-phenyl-butene, b.p. 120—130°C. Yield 85%.

Example 21

Preparation of 3-(4-bromophenyl)-3-phenyl-1-methylallylbromide

To a solution of 22.6 g (0.0787 mol) of 1-(4-bromophenyl)-1-phenylbutene in 800 ml of carbon tetrachloride 14.0 g (0.0787 mol) of N-bromosuccinimide was added. The mixture was heated to reflux temperature after the addition of 0.7 g of alpha, alpha-azobisbutyronitrile. After 3.5 hours all NBS had been consumed and the reaction mixture was cooled and filtered. 7.8 g of succinimide was separated and the volume of the filtrate was reduced to 50 ml by evaporation at 35°C. TLC of a sample on silica in diisopropyletherhexane (1:1) showed a new spot at Rf 0.62 (the starting olefin had Rf 0.55). The material was being used without further isolation or purification due to its high reactivity with nucleophilic agents.

Example 22

Preparation of 3-(N-phtalimido)-1-(4-bromophenyl)-1-phenylbutene (Method f)

A solution of crude 3-(4-bromophenyl)-3-phenyl-1-methylallylbromide (29 g, 0.08 mol) in 50 ml carbon tetrachloride was mixed with 15.0 g (0.08 mol) of N-potassiumpthalimide and 120 ml of anhydrous dimethylformamide. The mixture was stirred at 50°C for 14 hours.

Dilution with water (excess) and extraction of the product with diethylether gave 18 g of an oil after drying and evaporation of the solvent. Column chromatography on silica with diisopropylether as the eluent afforded the geometrical isomers: 2.6 g of the (Z)-form, $R_f = 0.30$, and 3.7 g of the (E)-form, $R_f = 0.26$ in diisopropylether-hexane (1:1).

Example 23

Preparation of (Z)-3-amino-1-(4-bromophenyl)-1-phenylbutene maleate

To a stirred solution of 0.43 g (0.001 mol) of (Z)-3-phtalimido-1-(4-bromophenyl)-1-phenylbutene in 30 ml of methanol 0.25 g (0.005 mol) of hydrazine hydrate was added at room temperature. In order to dissolve all the phtalimide 10 ml of carbon tetrachloride was added.The mxiture was stirred and heated at 60°C for 2 hours. After cooling the solvent was removed in vacuo and the residue was taken up in ether. The product was extracted with 3 x 50 ml of 0.5 M HCl, the combined aqueous layer was made alkaline with 10 M NaOH and extracted with 2 x 50 ml of ether. Drying and evaporation of the solvent gave 0.23 g of the title compound as an oil. The maleate had m.p. 174—176°C from ethanol. The UV spectrum in ethanol had $\lambda$ max 237 nm.

Elemental analysis: $C_{20}H_{20}BrNO_4$; Found: C 57.1, H 4.80, Br 20.3, N 3.10 and O 15.2%. Calculated: C 57.43, H 4.82, Br 19.10, N 3.35 and O 15.30%.

Example 24

(E)-3-amino-1-(4-bromophenyl)-1-phenylbutene oxalate was prepared from the corresponding phthalimide according to Example 23. M.p. 145—148°C.

Example 25

Preparation of (Z)-3-amino-1-(3-bromophenyl)-1-phenylbut-1-ene maleate (Method f)

To a stirred solution of 0.43 g (0.001 mol) of (Z)-3-phtalimido-1-(3-bromophenyl)-1-phenylbutene in 40 ml of methanol 0.35 g (0.007 mol) of hydrazine hydrate was added at room temperature. The mixure was heated under reflux for 2.5 hours. The solvent was evaporated and the residue was taken up in ether. Extraction with 3 x 25 ml of 0.5 M HCl followed by alkalization of the combined aqueous layer with 10 M NaOH and extraction with 2 x 50 ml of ether gave 0.19 g of residue after drying and evaporation of the solvent. The maleate was prepared from 15 ml ethylacetate — ethanol (2:1) to give 0.12 g (28%). M.p. 198—200°C.

Elemental analysis: $C_{20}H_{20}BrNO_4$: Found: C 56.3, H 4.7 and N 3.2%. Calculated C 57.43, H 4.82 and N 3.35%.

Example 26

(E)-3-amino-1-(3-bromophenyl)-1-phenylbutene hydrochloride was prepared from the corresponding phthalimide according to Example 25. M.p. 118—123°C.

Example 27

(Z)-3-amino-1-(4-bromophenyl)-1-(3-pyridyl)-butene oxalate was prepared from the corresponding phthalimide according to Example 25. M.p.

Example 28

Preparation of 3-(N-Phthalimido)-1-(4-bromophenyl)-1-phenylbutane

3-(4-bromophenyl)-1-methyl-3-phenylpropylamine as the free base (41.2 g, 0.136 mol) was dissolved in 350 ml of acetic acid. Phthalic anhydride (20.0 g, 0.136 mol) was added and the mixture was heated with stirring under reflux (bath temperature 120°C) for 2 hours. After cooling the solvent was evaporated in vacuo. The residue was shaken with a mixture of 800 ml of ether and 500 ml of

2 M NaOH. The ethereal layer was separated and washed with 100 ml I M hydrochloric acid. Drying and evaporation gave 49.5 g of a tan oil. Thin layer chromatography on silica in diisopropylether showed one spot of Rf 0.42. NMR showed a four proton multiplet at 7.7 ppm from TMS, characteristic of phthalimides. The material was used without further purification. Yield: 83%.

Example 29

Preparation of 3-(N-phthalimido)-1-(4-bromophenyl)-1-phenylbut-1-ene (Method g)

To a stirred solution of 24.6 g (0.057 mol) 3-(N-phthalimido)-1-(4-bromophenyl)-1-phenylbutane in 400 ml of carbon tetrachloride 10.0 g (0.057 mol) of N-bromosuccinimide was added. The mixture was stirred and 0.5 g of alpha, alpha-azoisobutyronitrile was added as radical initiator. Stirring was continued under reflux temperature for 2.5 hours. The reaction mixture was cooled and filtered. Upon evaporation of the solvent 32.4 g of a residue was obtained. NMR of a sample in $CDCl_3$ showed a double quartet at 5.0 ppm from TMS and a doublet at 6.6 ppm. TLC on silica in diisopropylether showed a spot with Rf 0.28, identical with the Rf-value of the material prepared according to Example 22.

The following examples illustrate how the compound of the present invention may be included in pharmaceutical preparations.

Example 30

Preparation of soft gelatin capsules

500 g of active substance were mixed with 500 g of corn oil, whereupon the mixture was filled in soft gelatin capsules, each capsule containing 100 mg of the mxiture (i.e. 50 mg of active substance).

Example 31

Preparation of soft gelatin capsules

500 g of active substance were mixed with 750 g of peanut oil, whereupon the mixture was filled in soft gelatin capsules, each capsule containing 125 mg of the mixture (i.e. 50 mg of active substance).

Example 32

Preparation of tablets

50 kg of active substance were mixed with 20 kg of silicic acid of the trade mark Aerosil. 45 kg of potato starch and 50 kg of lactose were mixed therewith and the mixture was moistened with a starch paste prepared from 5 kg of potato starch and distilled water, whereupon the mixture was granulated through a sieve. The granulate was dried and sieved, whereupon 2 kg of magnesium stearate was mixed into it. Finally the mixture was pressed into tablets each weighing 172 mg.

Example 33

Preparation of an emulsion

100 g of active substance were dissolved in 2500 g of peanut oil. From the solution thus obtained, 90 g of gum arabic, aroma and colouring agents (q.s.) and 2500 g of water an emulsion was prepared.

Example 34

Preparation of a syrup

100 g of active substance were dissolved in 300 g of 95% ethanol, whereupon 300 g of glycerol, aroma and colouring agents (q.s.) and 1000 ml of water were mixed therein. A syrup was obtained.

Example 35

Preparation of a solution

100 g of active substance were dissolved in 2000 g of polyoxyethylene sorbitane monooleate, whereupon flavouring agents and colouring agents (q.s.) and water to 5000 ml was mixed therein. A dropping solution was obtained.

Example 36

Preparation of effervescing tablets

100 g of active substance, 140 g of finely divided citric acid, 100 g of finely divided sodium hydrogen carbonate, 3.5 g of magnesium stearate and flavouring agents (q.s.) were mixed and the mixture was pressed into tablets each containing 100 mg of active substance.

Example 37

Preparation of a dropping solution

100 g of active substance were mixed with 300 g of ethanol, whereupon 300 g of glycerol, water to 1000 ml, aroma and flavouring agents (q.s.) and 0.1 N sodium hydroxide solution (to pH 4.5 to 5.5) was added while stirring. A dropping solution was obtained.

Example 38

Preparation of a sustained release tablet

200 g of active substance were melted together with 50 g of stearic acid and 50 g of carnauba wax. The mixture thus obtained was cooled and ground to a particle size of at most 1 mm in diameter. The mixture thus obtained was mixed with 5 g of magnesium stearate and pressed into tablets each weighing 305 mg. Each tablet thus contains 200 mg of active substance.

Pharmacological Evaluation

Depressions are considered to be connected with changes in the biochemical processes of the brain which processes control the mood. The nature of these biochemical processes are largely unknown but in depressive states there is evidence for a decreased activity of monoaminergic brain neurons. The monoamines, noradrenaline (NA), dopamine (DA) and 5-hydroxytryptamine (5-HT), are of great interest in this respect.

It has been demonstrated that NA, DA and 5-HT is localised in three different types on neurons and may function as transmittors in the central nervous system. The monoamines are stored in special structures, granules, situated in enlargements of the nerve endings, varicosities. The varicosity is separated from the effector neuron by a space, the synaptic cleft or spatium. As a result of a nerve stimulation the transmittor is released from the granule into the synaptic cleft and reaches the receptor of the effector neuron and generates a nerve impulse. After impulse generation the amines are inactivated by mainly two mechanisms: a re-uptake mechanism at the cell membrane and enzymatic conversion by catechol-O-methyltransferase to form methylated metabolites. There is also an inactivating enzyme within the varicosities, monoamine oxidase (MAO), that is stored in the mitochondria and inactivates the amines intracellularly.

When MAO-inhibitors are administered, an increased amount of transmittor substance becomes available for release at the nerve ending.

Another way of increasing the amine levels at the receptor is exerted by the tricyclic antidepressants. It has been shown that this type of compounds inhibits the re-uptake mechanism of NA and 5-HT, and the antidepressive action is assumed to be related to the uptake inhibition of NA and 5-HT.

It has been proposed, that some depressions are caused by deficiency in either one of the neuro-transmittors and some of deficiency in both.

An antidepressant effect should thus be obtained with compounds which are able to inhibit the re-uptake of one or both NA and 5-HT.

Pharmacological Methods

The test method described in Europ. J. Pharmacol. 17, 107, 1972. This method involves the measurement of the decrease in the uptake of $^{14}$C-5-hydroxytryptamine ($^{14}$C-5-HT) and $^{3}$H-noradrenaline ($^{3}$H—NA) in brain slices from mice after in vivo and in vitro administration of the test substance.

*Inhibition of the uptake of $^{14}$C-5-HT and $^{3}$H—NA in vitro and in vivo*

The test substances were administered intraperitoneally half an hour before the animals were killed. The midbrain was taken out, sliced and incubated in a mixture consisting of 0.2 nmole of $^{14}$C-5-HT, 0.2 nmole of $^{3}$H—NA and 11 $\mu$mole of glucose in 2 ml of Krebs Henseleit-buffer, pH 7.4 per 100 mg of brain slices. The incubation time was 5 minutes with 5 minutes of preincubation before the labelled amines were added. The slices were dissolved in Soluene (Trade Mark) and the amounts of radioactive amines taken up were determined by liquid scintillation. The doses producing 50 percent decrease of the active uptake ($ED_{50}$) of $^{14}$C-5-HT and $^{3}$H—NA were determined graphically from dose response curves. Active uptake is defined as that part of the radioactive uptake which is inhibited by a high concentration of cocaine.

In the vitro administration method slices of mouse midbrain were preincubated for 5 minutes with solution of the compound to be tested and then incubated as described above. The concentration producing 50 per cent inhibition of the active uptake $IC_{50}$ of $^{14}$C-5-HT and 3-H-NA was determined graphically from dose response curves.

The test results are given in Table I.

TABLE I

Inhibition of neutronal uptake of 5-hydroxytryptamine and noradrenaline in slices from mouse brain

| Compound | | | | | | | | Inhibition of the uptake in vitro $IC_{50}$ ($\mu M$) | | Inhibition of the uptake in vivo i.p. $ED_{50}$ ($\mu mole/kg$) | |
|---|---|---|---|---|---|---|---|---|---|---|---|
| | | | | | | | | NA | 5-HT | NA | 5-HT |
| Compounds of the invention | | | | | | | | | | | |
| 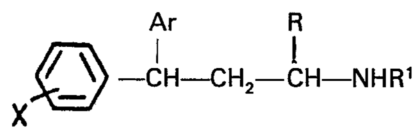 | Ar | Y | X | R | $R^1$ | isomer | salt[1] | | | | |
| *Code* | | | | | | | | | | | |
| CPK 163 | 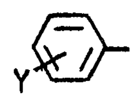 | 4-F | H | $CH_3$ | H | α | Ox | 0.54 | 0.28 | 32 | 32 |
| CPK 170 | „ | 4-F | H | $CH_3$ | H | β | HCl | 0.25 | 0.18 | 34 | 37 |
| CPK 185 | „ | 2-Br | H | $CH_3$ | H | α | Mal | 0.36 | 2.24 | 4.1 | 5.7 |
| CPK 197 | „ | 2-Br | H | $CH_3$ | H | β | Mal | 0.24 | 0.50 | 28 | >95 |
| CPK 155 | „ | 3-Br | H | $CH_3$ | H | α | Mal | 2.0 | 0.8 | 49 | >95 |
| CPK 184 | „ | 3-Br | H | $CH_3$ | H | β | Mal | 0.31 | 0.11 | 7.7 | 9.9 |
| CPK 165 | „ | 4-Br | H | $CH_3$ | H | α | Mal | 0.95 | 0.62 | 62 | 61 |
| CPK 171 | „ | 4-Br | H | $CH_3$ | H | β | Mal | 0.21 | 0.14 | 29 | 29 |

TABLE I cont.

| Compound | | | | | | | | Inhibition of the uptake in vitro $IC_{50}$ ($\mu M$) | | Inhibition of the uptake in vivo i.p. $ED_{50}$ ($\mu mole/kg$) | |
|---|---|---|---|---|---|---|---|---|---|---|---|
| | | | | | | | | NA | 5-HT | NA | 5-HT |
| Compounds of the invention | | | | | | | | | | | |
| 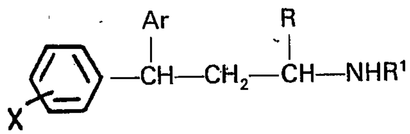 Code | Ar | Y | X | R | $R^1$ | isomer | salt[1] | | | | |
| CPK 187 | 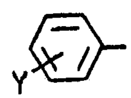 | 4-Br | H | $CH_3$ | $CH_3$ | $\alpha+\beta$ | Ux | 8.00 | 0.71 | >98 | 98 |
| CPK 195 | " | 4-$CF_3$ | H | $CH_3$ | H | $\alpha$ | Mal | 4.0 | 2.7 | >98 | 98 |
| CPK 199 | " | 4-$CF_3$ | H | $CH_3$ | H | $\beta$ | Mal | 2.25 | 0.76 | >98 | >98 |
| CPK 180 | " | 4-$CH_3O$ | H | $CH_3$ | H | $\alpha$ | Mal | 0.51 | 0.30 | 78 | 54 |
| CPK 198 | " | 4-$CH_3O$ | H | $CH_3$ | H | $\beta$ | Mal | 0.83 | 0.16 | 52 | 33 |
| FLA 615 | " | 4-F | 4-F | $CH_3$ | H | — | | 1.9 | 0.30 | 57 | 29 |
| FLA 619 | " | 3-$CH_3$ | 3-$CH_3$ | $CH_3$ | H | — | | 0.8 | 0.7 | 48 | 116 |
| FLA 614 | " | 4-$CH_3O$ | 4-$CH_3O$ | $CH_3$ | H | — | | 0.80 | 0.15 | 100 | 25 |

TABLE 1 cont.

| Compound | | | | | | | | Inhibition of the uptake in vitro $IC_{50}$ ($\mu M$) | | Inhibition of the uptake in vivo i.p. $ED_{50}$ ($\mu mole/kg$) | |
|---|---|---|---|---|---|---|---|---|---|---|---|
| | | | | | | | | NA | 5-HT | NA | 5-HT |
| Compounds of the invention | | | | | | | | | | | |
| 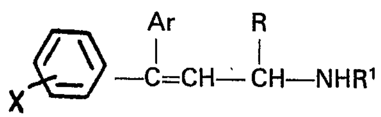 Code | Ar | Y | X | R | $R^1$ | isomer* | salt** | | | | |
| FLA 608 | 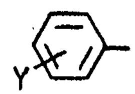 | 4-Br | 4-Br | $CH_3$ | H | — | HCl | 4.8 | 2.5 | 57 | 62 |
| FLA 611 | " | 4-$OCH_3$ | 4-$OCH_3$ | $CH_3$ | H | — | Fum | 1.7 | 0.4 | 108 | 63 |
| FLA 613 | " | 4-F | 4-F | $CH_3$ | H | — | Fum | 1.5 | 0.3 | 106 | 38 |
| FLA 618 | " | 3-$CH_3$ | 3-$CH_3$ | $CH_3$ | H | — | HCl | 2.4 | 1.3 | 56 | 93 |
| CPK 150 | " | 4-Br | H | $CH_3$ | H | Z | Mal | 2.0 | 3.0 | 96 | >96 |
| CPK 204 | " | 4-Br | H | $CH_3$ | H | E | Ox | 2.6 | 0.6 | 48 | 40 |
| CPK 217 | " | 3-Br | H | $CH_3$ | H | Z | Mal | 1.1 | 0.6 | 35 | 118 |
| CPK 215 | " | 3-Br | H | $CH_3$ | H | E | HCl | 1.3 | 0.7 | 24 | 96 |
| CPP 179 | 3-pyridyl | | 4-Br | $CH_3$ | H | Z | Ox | 1.6 | 0.2 | >89 | 89 |

TABLE I cont.

| Compound | | Inhibition of the uptake in vitro $IC_{50}$ ($\mu M$) | | Inhibition of the uptake in vivo i.p. $ED_{50}$ ($\mu mole/kg$) | |
|---|---|---|---|---|---|
| | | NA | 5-HT | NA | 5-HT |
| *Prior art compounds* | | | | | |
| 3,3-diphenyl-1-methylpropylamine fumarate | | 0.50 | 0.50 | 50 | 75 |
| N-methyl-3-(4-bromophenyl)-3-(3-pyridyl)-allylamine | E-isomer | 0.8 | 2.5 | 25 | 102 |
| | Z-isomer | 1.5 | 0.10 | >102 | 19 |
| Chloroimipramine | | 0.9 | 0.09 | 150 | 20 |
| Bromfeniramine | | 3.8 | 0.2 | 50 | 10 |

* Geometrical isomerism

** HCl = hydrochloride
Mal = maleate
Ox = oxalate
Fum = fumurate

The pharmacological tests show that the compounds are able to inhibit the uptake of noradrenaline and 5-hydroxytryptamine. A pronounced non-selective activity is shown for the compounds having codes CPK 170, CPK 171, CPK 180 and CPK 184. A pronounced selective activity on uptake of noradrenaline is seen in compounds CPK 185, CPK 215 and CPK 217 while a pronounced selective activity on uptake of 5-hydroxytryptamine is seen in compounds FLA 611, FLA 615, CPK 198 and CPK 204.

A strong non-selective activity is considered to be especially advantageous, as compounds having such activity may be employed in the treatment of depressions in which the neurotransmittor deficiency is unknown as well as in those cases wherein it is established that the deficiency pertains to both noradrenaline and 5-hydroxytryptamine.

**Claims**

1. A compound of the general formula

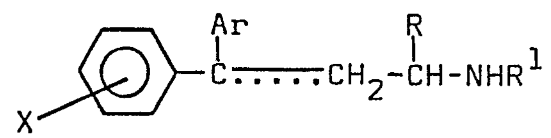

I

wherein the dotted line represents an optional double bond and Ar represents the group

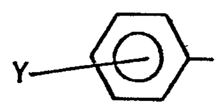

wherein Y is bound in the 2-, 3-, or 4-position and represents a lower alkyl or lower alkoxy group, a halogen, a trifluoromethyl group or an amino, mono- or di-lower alkylamino group, or Ar represents a pyridyl group bound in the 2-, 3-, 4-position, X represents hydrogen, R represents a lower alkyl group, and $R^1$ represents hydrogen or a lower alkyl group, wherein "lower" indicates a group having up to 3 carbon atoms; or a therapeutically acceptable acid addition salt thereof, in anhydrous or hydrated form.

2. A compound according to claim 1 of the formula

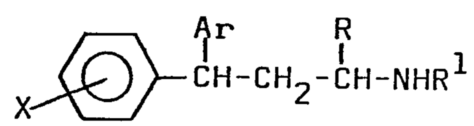

Ia

wherein Ar, X, R and $R^1$ have the meaning defined in claim 1 or a therapeutically acceptable salt thereof.

3. A compound according to claim 1 of the formula

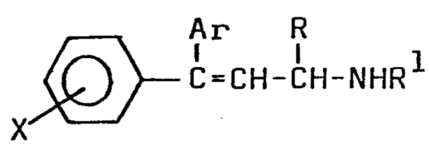

Ib

wherein Ar, X, R and $R^1$ have the meaning defined in claim 1, or a therapeutically acceptable salt thereof.

4. A compound according to any of claims 1, 2 and 3 in the form of
(a) a pure diastereomer,
(b) a pure geometrical isomer, or
(c) a pure optical enantiomer.

5. A compound according to any of claims 1, 2 and 3 wherein Ar represents the group

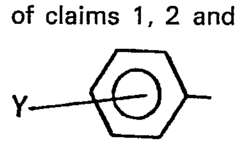

6. A compound according to claim 5 wherein Y represents F, Br, or $CH_3O$—.

7. A compound according to any of claims 1—6 wherein R is a methyl group.

8. A compound according to any of claims 1—7 wherein $R^1$ is hydrogen.

9. A compound according to claim 1 consisting of (β)-3-(4-fluorophenyl)-1-methyl-3-phenylpropylamine.

10. A compound according to claim 1 consisting of (β)-3-(4-bromophenyl)-1-methyl-3-phenylpropylamine.

11. A compound according to claim 1 consisting of (α)-3-(4-methoxyphenyl)-1-methyl-3-phenylpropylamine.

12. A compound according to claim 1 consisting of (β)-3-(3-bromophenyl)-1-methyl-3-phenylpropylamine.

13. A compound according to claim 1 consisting of (α)-3-(2-bromophenyl)-1-methyl-3-phenylpropylamine.

14. A compound according to claim 1 consisting of (E)-3-amino-1-(3-bromophenyl)-1-phenylbutene.

15. A compound according to claim 1 consisting of (Z)-3-amino-1-(3-bromophenyl)-1-phenylbutene.

16. A compound according to claim 1 consisting of (β)-3-(4-methoxyphenyl)-1-methyl-3-phenylpropylamine.

17. A compound according to claim 1 consisting of (E)-3-amino-1-(4-bromophenyl)-1-phenylbutene.

18. A pharmaceutical preparation which comprises as active ingredient a therapeutically effective amount of a compound of the formula

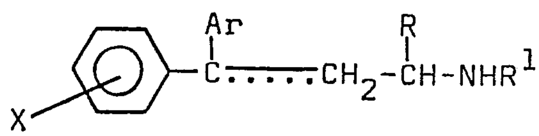

I

wherein the dotted line represents an optional double bond and Ar represents the group

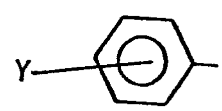

wherein Y is bound in the 2-, 3- or 4-position and represents a lower alkyl or lower alkoxy group, a halogen, a trifluoromethyl group or an amino, mono- or di-lower alkylamino gorup, or Ar represents a pyridyl group bound in the 2-, 3-, 4-position, X represents hydrogen, a lower alkyl or lower alkoxy group, a halogen, a trifluoromethyl group or an amino, mono- or di-lower alkylamino group, R represents a lower alkyl group, and $R^1$ represents hydrogen or a lower alkyl group, wherein "lower" indicates a group having up to 3 carbon atoms; or a therapeutically acceptable acid addition salt thereof, in anhydrous or hydrated form, in association with a pharmaceutically acceptable carrier.

19. A pharmaceutical preparation according to claim 18 which comprises as an active ingredient, a compound according to any of claims 1—17.

20. A compound of the formula

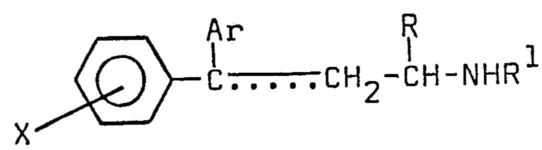

wherein Ar, X, R and $R^1$ have the meaning defined in claim 18, for use in the treatment of depression.

21. A compound as defined in any of claims 1—17 for use in the treatment of depression.

22. A compound of the formula

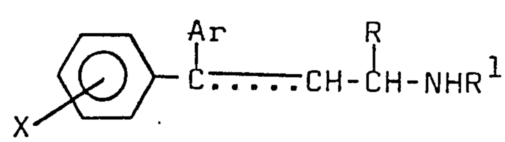

wherein Ar, X, R and $R^1$ have the meaning defined in claim 18, for use in the treatment of anxiety.

23. A compound as defined in any of claims 1—17 for use in the treatment of anxiety.

24. Compounds useful as intermediates in preparation of the compound defined in claim 1, characterized by the formula

(a)

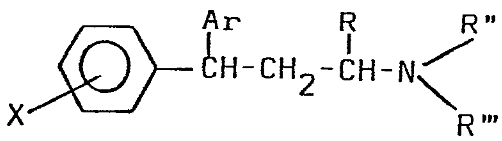

IX

wherein Ar, X and R have the meaning defined in claim 1 and R″ and R‴ are protective groups for the amino function;

(b)

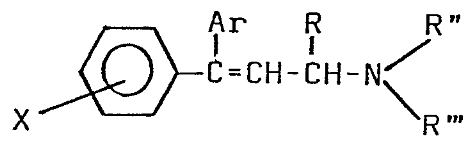

X

wherein Ar, X, and R have the meaning defined in claim 1 and R″ and R‴ are protective groups for the amino function.

**Revendications**

1. Composé, caractérisé en ce qu'il répond à la formule générale:

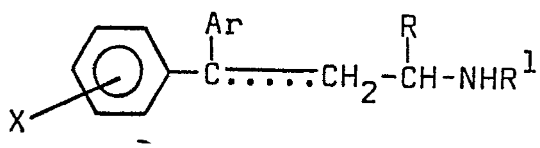

I

dans laquelle le trait en pointillé représente une double liaison facultative et Ar représente le radical

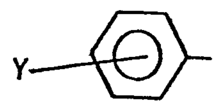

où Y est fixé en position 2,3 ou 4 et représente un radical alkyle inférieur ou un radical alcoxy inférieur, un atome d'halogène, un radical trifluorométhyle, ou un radical amino ou mono- ou di-alkyl (inférieur)-amino, ou bien Ar représente un radical pyridyle fixé en position 2, 3 ou 4, X représente un atome d'hydrogène, R représente un radical alkyle inférieur et $R^1$ représente un atome d'hydrogène ou un radical alkyle inférieur, le terme "inférieur" désignant un radical ayant jusqu'à 3 atomes de carbone, ou ses sels d'addition avec des acides thérapeutiquement acceptables, sous forme anhydre ou hydratée.

2. Composé selon la revendication 1, caractérisé en ce qu'il répond à la formule:

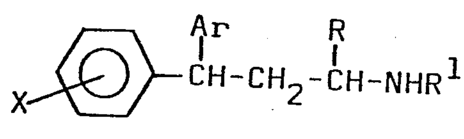

Ia

dans laquelle Ar, X, R et $R^1$ sont tels que définis dans la revendication 1, ou un sel d'addition de ce composé avec un acide thérapeutiquement acceptable.

3. Composé selon la revendication 1, caractérisé en ce qu'il répond à la formule:

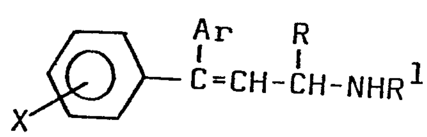

Ib

dans laquelle Ar, X, R et $R^1$ sont tels que définis dans la revendication 1, ou un sel d'addition de ce composé avec un acide thérapeutiquement acceptable.

4. Composé selon l'une quelconque des revendications 1 à 3, caractérisé en ce qu'il est sous la

forme (a) d'un diastéréoisomère pur, (b) d'un isomère géométrique pur ou (c) d'un énantiomère optique pur.

5. Composé selon l'une quelconque des revendications 1 à 3, caractérisé en ce que Ar représente le radical

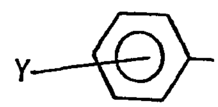

6. Composé selon la revendication 5, caractérisé en ce que Y représente F, Br ou $CH_3O$—.

7. Composé selon l'une quelconque des revendications 1 à 6, caractérisé en ce que R est un groupe méthyle.

8. Composé selon l'une quelconque des revendications 1 à 7, caractérisé en ce que $R^1$ est de l'hydrogène.

9. Composé selon la revendication 1, caractérisé en ce qu'il est la (bêta)-3-(4-fluorophényl)-1-méthyl-3-phényl-propylamine.

10. Composé selon la revendication 1, caractérisé en ce qu'il est la (bêta)-3-(4-bromophényl)-1-méthyl-3-phényl-propylamine.

11. Composé selon la revendication 1, caractérisé en ce qu'il est l'(alpha)-3-(4-méthoxyphényl)-1-méthyl-3-phényl-propylamine.

12. Composé selon la revendication 1, caractérisé en ce qu'il est la (bêta)-3-(3-bromophényl)-1-méthyl-3-phényl-propylamine.

13. Composé selon la revendication 1, caractérisé en ce qu'il est l'(alpha)-3-(2-bromophényl)-1-méthyl-3-phényl-propylamine.

14. Composé selon la revendication 1, caractérisé en ce qu'il est l'(E)-3-amino-1-(3-bromophényl)-1-phényl-butène.

15. Composé selon la revendication 1, caractérisé en ce qu'il est le (Z)-3-amino-1-(3-bromophényl)-1-phényl-butène.

16. Composé selon la revendication 1, caractérisé en ce qu'il est la (bêta)-3-(4-méthoxyphényl)-1-méthyl-3-phényl-propylamine.

17. Composé selon la revendication 1, caractérisé en ce qu'il est l'(E)-3-amino-1-(4-bromophényl)-1-phényl-butène.

18. Préparation pharmaceutique, caractérisée en ce qu'elle comprend, à titre de constituant actif, une quantité thérapeutiquement efficace d'un composé de formule

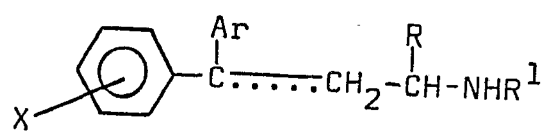

I

dans laquelle le trait en pointillé représente une double liaison facultative et Ar représente le radical

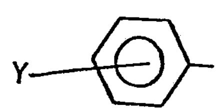

où Y est fixé en position 2, 3 ou 4 et représente un radical alkyle inférieur ou un radical alcoxy inférieur, un atome d'halogène, un radical trifluorométhyle, ou un radical amino ou mono- ou di-alkyl (inférieur)-amino, ou bien Ar représente un radical pyridyle fixé en position 2, 3 ou 4, X représente un atome d'hydrogène, un radical alkyle inférieur ou alcoxy inférieur, un atome d'halogène, un radical trifluorométhyle, ou un radical amino ou mono- ou di-alkyl (inférieur)-amino, R représente un radical alkyle inférieur et $R^1$ représente un atome d'hydrogène ou un radical alkyle inférieur, le terme "inférieur" désignant un radical ayant jusqu'à 3 atomes de carbone, ou ses sels d'addition avec des acides thérapeutiquement acceptables, sous forme anhydre ou hydratée.

19. Préparation pharmaceutique selon la revendication 18, caractérisée en ce qu'elle comprend à titre de constituant actif, un composé selon l'une quelconque des revendications 1 à 17.

20. Composé caractérisé en ce qu'il répond à la formule:

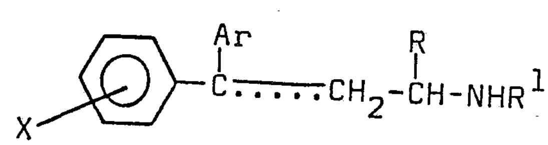

dans laquelle Ar, X, R et $R^1$ sont tels que déinis dans la revendication 18, et est destiné au traitement des dépressions.

21. Composé selon l'une quelconque des revendications 1 à 17, caractérisé en ce qu'il est destiné au traitement des dépressions.

22. Composé, caractérisé en ce qu'il répond à la formule:

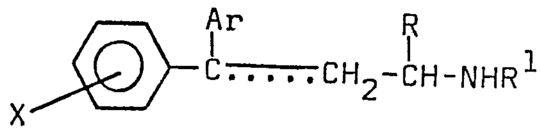

dans laquelle Ar, X, R et $R^1$ sont tels que définis dans la revendication 18, et est destiné au traitement de l'anxiété.

23. Composé selon l'une quelconque des revendications 1 à 17, caractérisé en ce qu'il est destiné au traitement de l'anxiété.

24. Composés utiles commes intermédiaires dans la préparation du composé défini dans la revendication 1, caractérisé en ce qu'il répond à la formule:

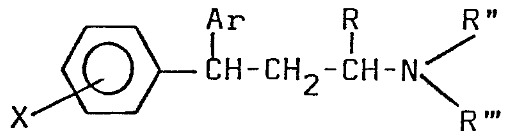
IX

dans laquelle Ar, X et R sont tels que définis dans la revendication 1, et R'' et R''' sont des groupes protecteurs de la fonction amino;

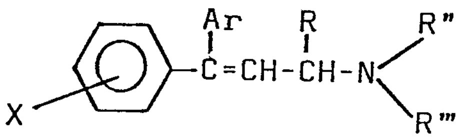
X

dans laquelle Ar, X et R sont tels que définis dans la revendication 1, et R'' et R''' sont des groupes protecteurs de la fonction amino.

**Patentansprüche**

1. Verbindung der allgemeinen Formel

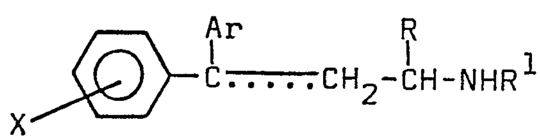
I

worin die gepunktete Linie eine eventuelle Doppelbindung bedeutet und Ar die Gruppe

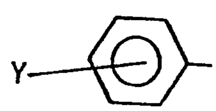

bedeutet, worin Y in der 2-, 3- oder 4-Stellung gebunden ist und eine niedermolekulare Alkylgruppe oder niedermolekulare Alkoxygruppe, ein Halogenatom, eine Trifluormethylgruppe oder eine Aminogruppe, Mono- oder Diniederalkylaminogruppe bedeutet, oder Ar eine in der 2-, 3- oder 4-Stellung gebundenen Pyridylgruppe bedeutet, X ein Wasserstoffatom bedeutet, R eine niedermolekulare Alkylgruppe bedeutet und $R^1$ Wasserstoff oder eine niedermolekulare Alkylgruppe bedeutet, worin "niedermolekular" bzw. "nieder" eine Gruppe mit bis zu 3 Kohlenstoffatomen bedeutet, oder ein therapeutisch verträgliches Säureadditionssalz derselben in wasserfreier oder hydratisierter Form.

2. Verbindung nach Anspruch 1 der Formel

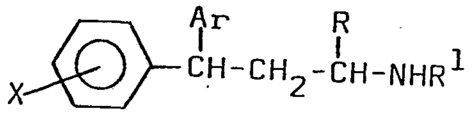

Ia

worin Ar, X, R und $R^1$ die in Anspruch 1 angegebene Bedeutung haben, oder ein therapeutisch verträgliches Salz derselben.

3. Verbindung nach Anspruch 1 der Formel

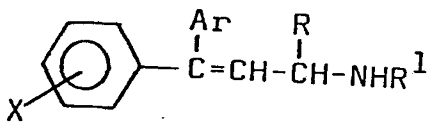

Ib

worin Ar, X, R und $R^1$ die in Anspruch 1 angegebene Bedeutung haben, oder ein therapeutisch verträgliches Salz derselben.

4. Verbindung nach einem der Ansprüche 1, 2 und 3 in der Form
a) eines reinen Diastereoisomeren,
b) eines reinen geometrischen Isomeren oder
c) eines reinen optischen Enantiomeren.

5. Verbindung nach einem der Ansprüche 1, 2 und 3, worin Ar die Gruppe

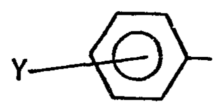

bedeutet.

6. Verbindung nach Anspruch 5, worin Y, F, Br der $CH_3O$— bedeutet.

7. Verbindung nach einem der Ansprüche 1 bis 6, worin R eine Methylgruppe ist.

8. Verbindung nach einem der Ansprüche 1 bis 7, worin $R^1$ Wasserstoff ist.

9. Verbindung nach Anspruch 1, bestehend aus ($\beta$)-3-(4-Fluorphenyl)-1-methyl-3-phenylpropylamin.

10. Verbindung nach Anspruch 1, bestehend aus ($\beta$)-3-(4-Bromphenyl)-1-methyl-3-phenylpropylamin.

11. Verbindung nach Anspruch 1, bestehend aus ($\alpha$)-3-(4-Methoxyphenyl)-1-methyl-3-phenylpropylamin.

12. Verbindung nach Anspruch 1, bestehend aus ($\beta$)-3-(3-Bromphenyl)-1-methyl-3-phenylpropylamin.

13. Verbindung nach Anspruch 1, bestehend aus ($\alpha$)-3-(2-Bromphenyl)-1-methyl-3-phenylpropylamin.

14. Verbindung nach Anspruch 1, bestehend aus (E)-3-Amino-1-(3-bromphenyl)-1-phenylbuten.

15. Verbindung nach Anspruch 1, bestehend aus (Z)-3-Amino-1-(3-bromphenyl)-1-phenylbuten.

16. Verbindung nach Anspruch 1, bestehend aus ($\beta$)-3-(4-Methoxyphenyl)-1-methyl-3-phenylpropylamin.

17. Verbindung nach Anspruch 1, bestehend aus (E)-3-Amino-1-(4-bromphenyl)-1-phenylbuten.

18. Pharmazeutisches Präparat, das als aktiven Bestandteil eine therapeutisch wirksame Menge einer Verbindung der Formel

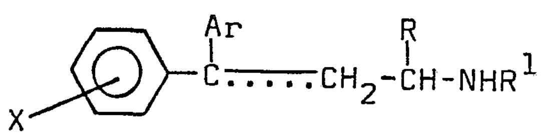

I

worin die gepunktete Linie eine eventuelle Doppelbindung bedeutet und Ar die Gruppe

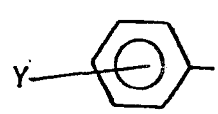

bedeutet, worin Y in der 2-, 3- oder 4-Stellung gebunden ist und eine niedermolekulare Alkylgruppe oder niedermolekulare Alkoxygruppe, eine Halogenatom, eine Trifluormethylgruppe oder eine Aminogruppe, Mono- oder Diniederalkylaminogruppe bedeutet, oder Ar eine in der 2-, 3- oder 4-Stellung gebundenen Pyridylgruppe bedeutet, X Wasserstoffatom, eine niedermolekulare Alkylgruppe oder niedermolekulare Alkoxygruppe, ein Halogenatom, eine Trifluormethylgruppe oder eine Aminogruppe, Mono- oder Diniederalkylaminogruppe bedeutet, R eine niedermolekulare Alkylgruppe bedeutet und $R^1$ Wasserstoff oder eine niedermolekulare Alkylgruppe bedeutet, worin "niedermolekular" bzw. "nieder" eine Gruppe mit bis zu 3 Kohlenstoffatomen bedeutet, oder ein therapeutisch verträgliches Säureadditionssalz derselben in wasserfreier oder hydratisierter Form in Verbindung mit einem pharmazeutisch verträglichen Trägermaterial enthält.

19. Pharmazeutisches Präparat nach Anspruch 18, das als aktiven Bestandteil ein Verbindung nach einem der Ansprüche 1 bis 17 enthält.

20. Verbindung der Formel

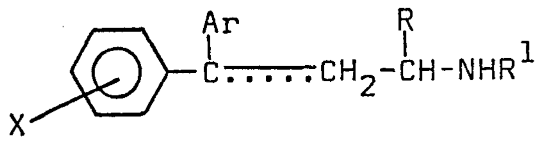

worin Ar, X, R und $R^1$ die in Anspruch 18 angegebene Bedeutung haben, für die Verwendung bei der Behandlung von Depressionen.

21. Verbindung nach einem der Ansprüche 1 bis 17 für die Verwendung bei der Behandlung von Depressionen.

22. Verbindung der Formel

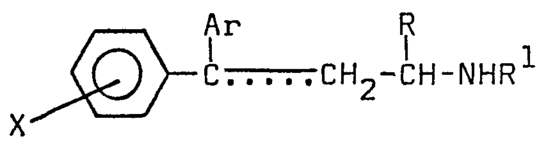

worin Ar, X, R und $R^1$ die in Anspruch 18 angegebene Bedeutung haben, für die Verwendung bei der Behandlung von Beklemmungen oder Angstzuständen.

23. Verbindung nach einem der Ansprüche 1 bis 17 für die Verwendug bei der Behandlung von Beklemmungen oder Angstzuständen.

24. Verbindungen, brauchbar als Zwischenprodukte bei der Herstellung der in Anspruch 1 definierten Verbindung, gekennzeichnet durch die Formel

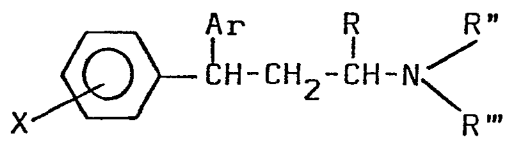

IX

worin Ar, X und R die in Anspruch 1 angegebene Bedeutung haben und R'' und R''' Schutzgruppen für die Aminofunktion sind, und durch die Formel

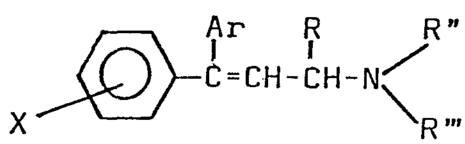

X

worin Ar, X und R die in Anspruch 1 angegebene Bedeutung haben und R'' und R''' Schutzgruppen für die Aminofunktion sind.